# EUROPÄISCHE PATENTSCHRIFT

(11) **EP 1 199 064 B1**
(45) Veröffentlichungstag und Bekanntmachung des Hinweises auf die Patenterteilung: **05.12.2007**
(21) Anmeldenummer: 01121600.9
(22) Anmeldetag: 11.09.2001
(51) Int. Cl.: A61Q 5/06, A61K 8/02, A61K 8/73, A61K 8/90, A61K 8/34

(54) **Haarbehandlungsmittel in Form eines festen und formstabilen Gels**
Hair care product in the form of a solid and dimensionally stable gel
Produit capillaire sous forme d'un gel solide et indéformable

(30) Priorität: 20.10.2000 DE 10051955
(43) Veröffentlichungstag der Anmeldung: 24.04.2002
(73) Patentinhaber: Wella Aktiengesellschaft, 64274 Darmstadt (DE)
(72) Erfinder: Birkel, Susanne, 64285 Darmstadt (DE); Lede, Michael, 63225 Langen (DE); Allwohn, Jürgen, 65558 Burgschwalbach (DE); Baecker, Sabine, 65428 Rüselsheim (DE); Krause, Thomas, 64297 Darmstadt (DE); Bayer, Angelika, 63857 Waldaschaff (DE)

(56) Entgegenhaltungen:
- EP-A- 0 651 990
- EP-A- 0 838 212
- EP-A- 0 923 931
- WO-A-00/78868
- WO-A-01/01950
- WO-A-98/00495
- WO-A-98/31336
- WO-A-02/098371
- DE-A- 3 834 354
- DE-A- 4 315 405
- FR-A- 2 595 944
- GB-A- 1 360 339
- GB-A- 2 227 660
- US-A- 4 901 795
- US-A- 5 002 934
- US-A- 5 263 223
- US-A- 5 593 680
- PATENT ABSTRACTS OF JAPAN vol. 008, no. 110 (C-224), 23. Mai 1984 (1984-05-23) & JP 59 025873 A (NIPPON SEKIYU KK), 9. Februar 1984 (1984-02-09)

## Beschreibung

Gegenstand der Erfindung ist ein Haarbehandlungsmittel in Form eines festen und formstabilen Gels auf Basis einer Carrageenan enthaltenden wässrigen Grundlage.

Um dem menschlichen Haar Festigung und Halt zu geben oder um eine erstellte Frisur zu stabilisieren, werden Haarbehandlungsmittel u.a. in Form von verdickten Präparaten wie z.B. Gelen eingesetzt. Derartige Produkte haben eine hochviskose, aber dennoch fliessfähige Konsistenz und sind üblicherweise in Kunststofftuben verpackt, aus denen sie bei Anwendung in Form eines Gelstrangs herausgedrückt werden. Sie enthalten in der Regel eine Kombination aus einem Gelbildner wie z.B. Polyacrylaten (Carbomere) und einem haarfestigenden Polymer wie z.B. Polyvinylpyrrolidon. Die für diese Zwecke üblicherweise verwendeten kosmetischen, haarfestigenden Polymere zeigen in den herkömmlichen wässrigen oder wässrig-alkoholischen Gelen gute Festigungseigenschaften, die nach der Anwendung die Haare in Form halten und festigen und die erstellte Frisur stabilisieren.

Nachteilig an herkömmlichen Haargelen ist, dass sie zerfliessen und nicht formstabil sind und es nicht möglich ist, sie bereits bei der Herstellung in festen, diskreten, für eine Anwendung ausreichenden Stücken zu portionieren. Auch ist eine dreidimensionale Gestaltbarkeit und die Bereitstellung von attraktiven äußeren Formen wie Würfeln, Kugeln etc. nicht möglich. Dünnflüssige Gele oder thixotrope Gele, die sich unter Druck verflüssigen, können zudem bei der Anwendung von den Händen oder den Haaren ablaufen. Ein weiterer Nachteil von typischen Carbomer-Gelen ist, dass sie nur in einem begrenzten pH-Bereich viskositätsstabil sind. Dies bedeutet zum einen einen erhöhten Aufwand bei der Produktion, indem bestimmte pH-Bereiche eingehalten werden müssen und zum anderen Inkompatibilitäten mit weiteren Inhaltsstoffen, welche ihrerseits nur bei anderen pH-Bereichen eingesetzt werden können.

Aus der EP 0 923 931 sind feste kosmetische Mittel bekannt, welche mindestens 2% kappa-Carrageenan in Kombination mit bestimmten Hydrokolloiden auf natürlicher Basis enthalten und im wesentlichen als Körperpflegestifte (Sticks, Lippenstifte) eingesetzt werden. Bei diesen Mitteln sind relativ hohe Mengen an Carrageenan erforderlich, um die notwendige Festigkeit zu erreichen. Derartige Mittel sind zur Haarbehandlung nicht geeignet, da sich aufgrund des hohen Carrageenangehaltes (größer 2 Gew.%) unschöne, sichtbare Rückstände auf dem Haar bilden können. Bei Herabsetzung der Carrageenanmenge ist eine ausreichende Festigkeit des Produktes nicht mehr gegeben.

Die der Erfindung zugrunde liegende Aufgabe bestand darin, ein Haarbehandlungsmittel zur Verfügung zu stellen, welches die Haare ausreichend festigt, bereits bei der Herstellung in stabilen, dreidimensionalen Formen portionierbar ist, gleichzeitig aber noch gut mit den Händen verreibbar oder auf dem Haar verteilbar ist, in einem weiten pH-Bereich viskositätsstabil ist und keine unerwünschten sichtbaren Rückstände auf dem Haar bildet.

Es wurde nun gefunden, dass die Aufgabe gelöst wird durch ein Haarbehandlungsmittel in Form eines festen und formstabilen Gels gemäß Anspruch 1.

Fest und formstabil im Sinne der Erfindung sind insbesondere solche Zusammensetzungen, für die der Widerstand gegen Kompression unter Normbedingungen (20°C, 65% rel. Luftfeuchte) mindestens 0,15 N, vorzugsweise 0,30 bis 2,0 N, besonders bevorzugt 0,40 bis 1,5 N beträgt, gemessen durch Penetration des festen Gels mit einem zylindrischen Stempel eines Durchmessers von 8 mm und wobei die Penetration mit einer Geschwindigkeit von 0,5 mm/sec bis zu einer Kompressionstiefe von 1 mm erfolgt und anschließend der Stempel mit einer Geschwindigkeit von 0,5 mm/s wieder zurückgeführt wird. Die Formstabilität des Gels besteht mindestens bei Raumtemperatur (20°C) und darunter, vorzugsweise bei Temperaturen bis 30°C, besonders bevorzugt bei Temperaturen bis 35°C.

Carrageenan und ein synthetisches haarfestigendes Polymer sind in einer solchen Menge enthalten, dass das Mittel bei Raumtemperatur in Form eines festen, formstabilen Gels vorliegt. Normalerweise ist eine Carrageenanmenge von mindestens etwa 3 Gew.% in Wasser erforderlich, um zu einem festen und formstabilen Gel zu gelangen, was für ein Haarbehandlungsmittel unakzeptabel ist, da es zu unerwünschten Rückständen auf dem Haar führt. Oberraschenderweise wurde gefunden, dass es möglich ist, Haarbehandlungsmittel in fester Gelform auf Basis von Carrageenan herzustellen, wobei die Carrageenanmenge soweit reduziert ist, dass die unerwünschte Rückstandsbildung auf dem Haar nicht auftritt. Die erforderliche Festigkeit des Gels kann dabei erreicht werden, indem mindestens ein haarfestigendes Polymer und/oder mindestens 15 Gew.% mindestens eines ein- oder mehrwertigen Alkohols und/oder mindestens ein Calcium-oder Kaliumionen enthaltendes Salz zugesetzt werden.

Die erfindungsgemäßen Zusammensetzungen zeigen eine ausreichende Festigkeit des Gels bereits mit geringen Carrageenanmengen von 0.5 bis kleiner als 2 Gew.%. Hierdurch ist es möglich, die Zusammensetzungen zur Haarbehandlung, insbesondere bei Zusatz von haarfestigenden Polymeren als Haarstylingmittel zur Haarfestigung einzusetzen, da das Problem den unerwünschten Rückstandsbildung gelöst ist.

### Carrageenan

Erfindungsgemäße Gelbildner sind kappa-Carrageenan oder ein kappa-Carrageenan enthaltendes Carrageenan-Gemisch. Besonders gut geeignet ist beispielsweise ein Carrageenan mit mittlerem Molekulargewicht wie Sea Kem® CM 611 der Firma FMC Corporation. Der Gelbildner wird in einer solchen Menge eingesetzt, dass das Mittel bei Raumtemperatur (20°C) in Form eines festen, formstabilen Gels vorliegt. Erfindungsgemäße Mengen sind von 0,5 bis kleiner 2 Gew.%. Die Festigkeit kann sich dabei teilweise erst nach einiger Zeit, d.h. im Verlaufe von zwei bis drei Tagen einstellen. Die Verfestigung des Gels kann aber dadurch beschleunigt werden, dass der Gelbildner zunächst in Wasser gegebenenfalls unter Erwärmen auf etwa 80°C gelöst und anschließend schnell mittels zusätzlicher, externer Kühlung auf mindestens 50 bis 55°C oder darunter abgekühlt wird.

### Calcium- und Kaliumionen

Die Calcium- und/oder Kaliumionen werden in Form von wasserlöslichen Salzen, z.B. den Halogeniden, Sulfaten etc., von denen die Chloride bevorzugt sind, eingesetzt, wobei die Menge der Salze vorzugsweise 0,2 bis 1 Gew. %, besonders bevorzugt 0,4 bis 0,8 Gew.% beträgt. Kaliumionen sind bevorzugt, da sich hiermit klarere, ungetrübte Gele herstellen lassen.

### Haarfestigende Polymere

Es hat sich gezeigt, dass feste Gele aus reinem Carrageenan eine gewisse haarfestigende Wirkung besitzen auch ohne zusätzliche, übliche haarfestigende Polymere. Es sind für eine ausreichende Haarfestigung aber relativ hohe Carrageenanmengen erforderlich, was zu der unerwünschten Nebenwirkung von sichtbaren Rückständen auf dem Haar führt. Bei einer Absenkung der Carrageenanmenge gehen sowohl die ausreichende Haarfestigung als auch die Festigkeit und die Formstabilität des Gels verloren. Das erfindungsgemäße Mittel kann daher mindestens ein zusätzliches, synthetisches oder natürliches haarfestigendes Poymer enthalten.

Das zusätzliche haarfestigende Polymer liegt vorzugsweise in einer Menge von 0,1 bis 30 Gew.%, besonders bevorzugt von 0,5 bis 15 Gew.% vor. Das haarfestigende Polymer kann nichtionisch, anionisch, zwitterionisch oder amphoter sein. Besonders bevorzugt sind Polymere, welche keine kationischen Gruppen enthalten, d.h. anionische, nichtionische und amphotere Polymere. Unter synthetischen Polymeren werden solche Polymere verstanden, welche rein synthetischen, nicht natürlichen Ursprungs sind, insbesondere solche, die durch radikalische Polymerisation aus ethylenisch ungesättigten Monomeren oder durch Polykondensation herstellbar sind. Bevorzugt sind insbesondere solche Polymere, die eine ausreichende Löslichkeit oder Dispergierbarkeit in Wasser, Alkohol oder Wasser/Alkohol-Gemischen besitzen, um in dem erfindungsgemäßen Mittel auf wässriger Basis in gelöster oder homogen dispergierter Form vorzuliegen. Unter haarfestigenden Polymeren werden erfindungsgemäß solche Polymere verstanden, die bei Anwendung in 0,01 bis 5%iger wässriger, alkoholischer oder wässrigalkoholischer Lösung in der Lage sind, auf dem Haar einen Polymerfilm abzuscheiden und das Haar zu festigen.

Besonders bevorzugt werden in dem erfindungsgemäßen Gel filmbildende, haarfestigende, nichtionische, anionische oder amphotere Polymere eingesetzt. Geeignete nichtionische Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die z.B. unter den Handelsbezeichnungen Akypomine® P 191 von der Firma CHEMY, Emmerich, oder Sepigel® 305 von der Firma Seppic vertrieben werden; Polyvinylalkohole, die z.B. unter den Handelsbezeichnungen Elvanol® von Du Pont oder Vinol® 523/540 von der Firma Air Products vertrieben werden sowie Polyethylenglykol/Polypropylenglykol Copolymere, die z.B. unter den Handelsbezeichnungen Ucon® der Union Carbide vertrieben werden. Besonders bevorzugt sind Polyvinylpyrrolidon, Polyvinylcaprolactam und deren Copolymere mit mindestens einem weiteren nichtionischen Monomer, insbesondere Polyvinylpyrrolidon/ Vinylacetat Copolymere.

Geeignete anionische haarfestigende Polymere sind synthetische Homo- oder Copolymere aus Säuregruppen enthaltenden Monomereinheiten, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und - OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin, Monoethanolamin oder Tetrahydroxypropylethylendiamin und Ammoniak, NaOH und andere. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind z.B. Acrylsäure, Methacrylsäure, Crotonsäure, Maleinsäure bzw. Maleinsäureanhydrid oder deren Monoester.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkyl-gruppen sind.

Geeignete Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere bevorzugte anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere sowie Copolymere aus Acryl- oder Methacrylsäure und Acryl- oder Methacrylsäurealkylestern, wobei die Alkylgruppen vorzugsweise 1 bis 7 C-Atome enthalten.

Geeignete haarfestigende amphotere Polymere sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester, wie sie zum Beispiel unter dem Handelsnamen Amphomer^{®} oder Amphomer^{®} LV-71 der Firma NATIONAL STARCH, USA erhältlich sind. Weitere Beispiele für als Komponente (B) geeignete Copolymere mit Säuregruppen sind Copolymere von Acrylsäure, Methylacrylat und Methacrylamidopropyltrimethylammoniumchlorid (INCI-Bezeichnung: Polyquaternium-47), wie sie beispielswiese von der Firma Calgon unter der Handelsbezeichnung Merquat^{®} 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43), wie sie beispielsweise von der Firma Societe Francaise Hoechst unter der Handelsbezeichnung Bozequat^{®} 4000 vertrieben werden. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

### Wässrige Grundlage

Das erfindungsgemäße Gel wird bevorzugt in einer wässrigen Grundlage konfektioniert. Hierunter wird entweder ein rein wässriges oder ein wässrig-alkoholisches Medium mit vorzugsweise bis zu 40 Gew.% Alkoholen verstanden. Als Alkohole können insbesondere die für kosmetische Zwecke üblicherweise verwendeten niederen ein- oder mehrwertigen Alkohole mit 1 bis 5 Kohlenstoffatomen wie z.B. Ethanol, Isopropanol, Ethylenglykol, Glycerin und Propylenglykole, insbesondere 1,2-Propylenglykol, enthalten sein. Typische Wassergehalte sind 55 bis 95, bevorzugt 65 bis 80 Gew.%, typische Alkoholgehalte sind 0 bis 30, vorzugsweise 1 bis 25 Gew.%. Bei Alkoholgehalten über 40 Gew.% besteht die Gefahr, dass das Carrageenan ausfällt.

Besonders vorteilhaft ist die Verwendung von mindestens 15 Gew.% Alkohol, da hierbei eine ausreichende Festigkeit und Formbeständigkeit des Gels bereits mit Carrageenanmengen von weniger als den sonst mindestens erforderlichen 2,5 bis 3 Gew.%, insbesondere mit weniger als 2 Gew.% erreicht werden können. Außerdem ist bei Verwendung von mindestens 15 Gew.% Alkohol ein zusätzliches Konservierungsmittel nicht unbedingt erforderlich.

Ein besonderer Vorteil des erfindungsgemäßen Gels ist die Viskositätsstabilität über einen weiten pH-Bereich von 1 bis 14. Besonders bevorzugt ist der pH-Bereich zwischen 2,5 und 8.

### Konservierungsmittel

Da das für das erfindungsgemäße Gel einzusetzende Carrageenan ein Polymer natürlichen Ursprungs auf Saccharidbasis ist, sind besondere Anforderungen an die Konservierung des erfindungsgemäßen Mittels zu stellen, um eine längere Haltbarkeit zu gewährleisten. Als besonders geeignete Konservierungsmittel haben sich Parabene, beispielsweise Methylbaraben erwiesen. Bei Ethanolgehalten um etwa 15 Gew.% und darüber ist ein zusätzliches Konservierungsmittel nicht unbedingt erforderlich.

### Zucker

Zur Verbesserung der Klarheit und Transparenz des erfindungsgemäßen Mittels ist es zweckmässig, dass zusätzlich mindestens ein Zucker enthalten ist. Geeignete Zucker sind insbesondere Mono- und Disaccharide wie z.B. Glukose, Galaktose, Fructose, Maltose, Lactose oder Saccharose. Typische Einsatzmengen sind 0,01 bis 5, vorzugsweise 0,05 bis 1 Gew.%. Bevorzugt werden fertige Mischungen von Carrageenan und Zucker wie z.B. der Rohstoff Seakem CM 611, bei dem es sich um eine Mischung von Carrageenan und Dextrose handelt.

### Zusätze für bessere Verreib- und Verteilbarkeit

Zur Verbesserung der Verreibarkeit in den Händen bzw. zur Verbesserung der Verteilbarkeit auf dem Haar oder zur weiteren Optimierung der Konsistenz enthält das erfindungsgemäße Mittel vorzugsweise weitere Verdicker oder Gelbildner. Hierfür geeignet sind z.B. Carboxyvinylpolymere, insbesondere Polyacrylate wie z.B. die verschiedenen Carbopol-Typen, außerdem Polyglykole, Cellulosederivate, insbesondere Hydroxyalkylcellulosen sowie anorganische Verdicker wie z.B. natürliche oder synthetische Bentonite. Typische Einsatzkonzentrationen der zusätzlichen Gelbildner und Verdicker sind von etwa 0,2 bis 10,0 Gew.%, vorzugsweise 1 bis 5 Gew.%.

Geeignete Substanzen, welche die Verreibbarkeit des Gels oder die Verteilbarkeit des Gels auf Haaren erleichtern, sind Xanthan Gum und Cellulosederivaten, wie sie in der EP 0 923 931 beschrieben werden. Hierbei handelt es sich um in heissem Wasser lösliche Hydrokolloide, insbesondere um Carboxymethylcellulose und Hydroxyethylcellulose.

Überraschenderweise wurde gefunden, dass eine besonders gute Verreibarkeit und Verteilbarkeit von festen Gelen auf Carrageenan-Basis erzielt wird bei Verwendung von amphiphilen Assoziativverdickern. Geeignete amphiphile Assoziativverdicker sind nichtionische Polymere, welche sowohl hydrophile als auch hydrophobe Gruppen enthalten. Assoziativverdicker sind wasserlösliche Polymere und haben tensidartige hydrophobe Bestandteile, welche in der Lage sind, sich in einem hydrophilen, insbesondere wässrigen Medium sowohl mit sich selbst als auch mit anderen hydrophoben Stoffen zu assoziieren, d.h. in Wechselwirkung zu treten. Durch das daraus resultierende assoziative Netzwerk wird das Medium verdickt oder geliert. Typischerweise werden Assoziativverdicker hergestellt durch Polymerisation von Polyethylenoxid-Prepolymeren und mindestens zweifach funktionellen, polykondensierbaren Stoffen wie z.B. Isocyanaten, wobei Mono- oder Diole mit großen Aryl-, Alkyl- oder Aryl/Alkyl-Gruppen eingebaut werden, um die hydrophobe Modifikation bereitzustellen. Bevorzugte Assoziativverdicker sind daher hydrophob modifizierte Polyalkylenglykole. Hierbei wird der hydrophile Bestandteil durch Polyoxyalkyleneinheiten, vorzugwseise Polyoxyethylenaber auch Polyoxypropyleneinheiten oder deren Gemisch gebildet. Der hydrophobe Bestandteil wird vorzugsweise aus Kohlenwasserstoffgruppen, z.B langkettigen Alkyl-gruppen, Alkylaryl- oder Arylalkylgruppen gebildet.

Besonders bevorzugte Assoziativverdicker sind hydrophob modifizierte Aminoplast-Polyether Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethern sowie einfach funktionellen Verbindungen mit hydrophoben Gruppen. Geeignete Aminoplaste sind der Figur 1 der WO 96/40815 zu entnehmen. Besonders bevorzugt sind die Glycolurilderivate der Formel X der WO 96/40815. Geeignete Alkylenpolyether sind der Figur 2 der WO 96/40815 zu entnehmen. Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815.

Erfindungsgemäß geeignete Assoziativverdicker sind ausgewählt aus Polymeren der allgemeinen Formel (I) 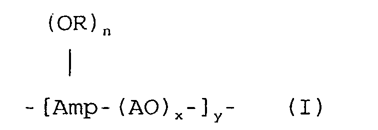 wobei Amp ein Aminoplastmonomer oder den Rest eines Aminoplastoligomers oder eines Aminoplastpolymers bedeutet, AO für eine Alkylenoxidgruppe steht, R für Wasserstoff, C1-C4-Alkyl oder C1-C4-Acyl steht, x und y Zahlen größer 1 sind und n eine positive Zahl ist, die der Anzahl der freien Valenzen von Amp entspricht. Besonders bevorzugt sind die Reaktionsprodukte der säurekatalysierten Polykondensation von (a) Glykolurilen der allgemeinen Formel (II), 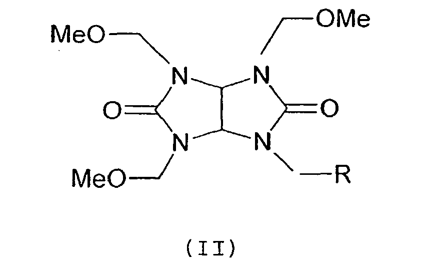 wobei R für H oder vorzugsweise für OMe steht mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind. Besonders bevorzugtes Glykoluril ist 1,3,4,6-Tetramethoxymethylglycoluril.

Geeignete Assoziativverdicker sind solche mit den INCI-Bezeichnungen Polyether-1, PEG-180/Octoxynol-40/TMMG Copolymer und PEG-180/Laureth-50/TMMG Copolymer und werden von der Firma Süd-Chemie vertrieben unter den Handelsbezeichnungen Pure-Thix^{®} HH, L, M oder TX.

### Optionale Zusatzbestandteile

Das erfindungsgemäße Mittel kann darüber hinaus die für Haarbehandlungsmittel üblichen Zusatzbestandteile enthalten, z.B Netzmittel oder Emulgatoren in einer Menge von 0,1 bis 15 Gew.%; Feuchthaltemittel; Parfümöle in einer Menge von 0,1 bis 0,5 Gew.%; bakterizide und fungizide Wirkstoffe wie zum Beispiel 2,4,4-Trichlor-2-hydroxydiphenylether oder Methylchlorisothiazolion, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie zum Beispiel Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe in einer Menge von etwa 0,01 bis 2 Gew.%; Glanzgeber, Vitamine, Weichmacher, Kämmbarkeitsverbesserer und rückfettende Agenzien.

### Herstellverfahren

Das erfindungsgemäße Mittel kann hergestellt werden, indem zunächst in einem wässrigen Lösungsmittel das Carrageenan (A) sowie gegebenenfalls weitere haarkosmetische Wirk- und Hilfsstoffe gelöst werden, wobei die Menge des Gelbildners sowie Art und Mengen der weiteren haarkosmetischen Wirk- und Hilfsstoffe so gewählt sind, dass sich ein formstabiles, festes Gel ausbilden kann. Falls der Gelbildner oder die Zusatzstoffe bei Raumtemperatur nicht vollständig löslich sind, wird zum Lösen der Stoffe erwärmt, z.B. auf etwa 40 bis 80°C. Anschließend wird die Lösung solange stehen gelassen, bis das Gel fest wird. Dieser Prozess wird vorteilhafterweise durch zusätzliche externe Abkühlung auf mindestens 50 bis 55°C oder darunter beschleunigt.

Das erfindungsgemäße Mittel zeichnet sich dadurch aus, dass es in bei Raumtemperatur (20 °C) nicht zerfließende, stabile, dreidimensionale Formen portionierbar und gestaltbar hergestellt werden kann. Geeignete dreidimensionale Formen sind z.B. Würfel, Quader, Kugeln, Eier, Herzen, Buchstaben, Logos, Tierkörper, Sternzeichen oder sonstige Mini-Skulpturen. Diese Formen können entweder hergestellt werden, indem eine erwärmte, noch fließfähige Zusammensetzung in eine entsprechende Form gegossen und abgekühlt wird, oder indem die gewünschte Form aus einem größeren Block mittels eines geeigneten Schneidwerkzeugs ausgeschnitten oder mittels eines geeigneten Stanzwerkzeugs ausgestanzt wird.

Ein bevorzugtes Verfahren zur Formgebung besteht darin, dass in eine geeignete Vorlage mit einem Stempel die gewünschte Form gedrückt wird. Die Vorlage kann aus einem Stärkematerial bestehen, z.B. aus konditioniertem Stärkepulver, hydrophobem Stärkepulver oder aus einem Teig aus Getreidemehl, z.B. aus Maismehl. Dann wird die noch flüssige Gelmasse in die Formen aus Stärkepulver gegossen. Nach einer für die Verfestigung der Gelmasse ausreichenden Standzeit wird das Stärkepulver entfernt, z.B. durch Sieben, Blasen, Waschen oder Herausnehmen der festen Formen. Vorzugsweise erfahren die Artikel noch eine anschließende Oberflächenbehandlung zur Erzeugung einer glänzenden Oberfäche und zur Vermeidung eines Aneinanderklebens. Hierzu können die Artikel kurz mit Dampf besprüht oder mit Glycerin oder mit Silikonöl abgespült und anschließend getrocknet werden. Zusätzlich können die Artikel an der Oberfläche mit Fetten oder Wachsen, z.B. mit Bienenwachs überzogen werden.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

Die in den nachfolgenden Beispielrezepturen genannten Bestandteile werden jeweils unter Erwärmen auf ca. 70 °C in dem jeweiligen Lösungsmittel gelöst. In einem ersten Verfahren zur Formgebung wird anschließend durch Stehenlassen bis auf Raumtemperatur abgekühlt, wobei sich die Gele verfestigen. Die festen Gele werden portioniert, indem sie mit einem Messer in formstabile Würfel oder Quader geschnitten werden.

In einem alternativen, zweiten Verfahren zur Formgebung wird eine Schale mit hydrophobem Stärkepulver (Dry Flo^{®} PC von National Starch) gefüllt. In das Stärkepulver werden runde Vertiefungen eingedrückt. In die Vertiefungen wird das noch flüssige Gel gefüllt. Das Gel wird abkühlen gelassen. Nach der Verfestigung werden die festen Gelpellets aus dem Stärkepulver herausgenommen, abgesiebt und mit Glycerin oder Silikonöl (Baysilon^{®} PD 5) abgespült. Zur weiteren Glanzerhöhung können die Gelpellets mit Bienenwachs überzogen werden. Die festen Gelartikel waren auch bei zweiwöchiger Lagerung bei 40°C formstabil.

### Beispiel 1

| | |
|---|---|
| 1,5 g | Sea Kem^{®} CM 611 (Carrageenan und Dextrose, FMC Corp.) |
| 2,5 g | Luviset^{®} CA 66 (Vinylacetat/Crotonsäure Copolymer, BASF) |
| 0,26 g | Aminomethylpropanol (95%ig) |
| 2,5 g | Pure Thix^{®} TX 1442 (Polyether-1, Süd-Chemie, United Catalysts) |
| 20 g | Ethanol |
| ad 100 g | Wasser |

### Beispiel 2: Gel mit leichter Festigung

| | |
|---|---|
| 1,5 g | Sea Kem^{®} CM 611 (Carrageenan und Dextrose, FMC Corp.) |
| 2,0 g | Spezialschellack SSB 63 HE-N (Stroever GmbH & Co. KG) |
| 2,5 g | Pure Thix^{®} TX 1442 (Polyether-1, Süd-Chemie, United Catalysts) |
| 19,0 g | Ethanol |
| 0,3 g | Parfüm |
| 0,2 g | PEG-40 Hydrogenated Castor Oil |
| 0,2 g | PPG-1-PEG9 Lauryl Glycol Ether |
| 0,5 g | PEG-25 PABA |
| ad 100 g | Wasser |

### Beispiel 3: Gel mit starker Festigung

| | |
|---|---|
| 1,9 g | Sea Kem^{®} CM 611 (Carrageenan und Dextrose, FMC Corp.) |
| 3,5 g | Octylacrylamide/Acrylates/Butylaminoethyl-methacrylate Copolymer (Amphomer^{®}) |
| 0,6 g | Aminomethylpropanol 95%ig |
| 0,1 g | Carbomer |
| 0,95 g | NaOH, 5%ig |
| 19,0 g | Ethanol |
| 0,3 g | Parfüm |
| 0,2 g | PEG-40 Hydrogenated Castor Oil |
| 0,2 g | PPG-1-PEG9 Lauryl Glycol Ether |
| 0,3 g | Panthenol |
| ad 100 g | Wasser |

### Beispiel 4: Schnell trocknendes Gel

| | |
|---|---|
| 1,4 g | Sea Kem^{®} CM 611 (Carrageenan und Dextrose, FMC Corp.) |
| 2,5 g | Vinylacetat/Crotonat Copolymer (Luviset^{®} CA 66) |
| 0,27 g | Aminomethylpropanol 95%ig |
| 0,2 g | Hydroxyethylcellulose |
| 29,0 g | Ethanol |
| 0,3 g | Parfüm |
| 0,4 g | PEG-40 Hydrogenated Castor Oil |
| ad 100 g | Wasser |

### Beispiel 5: Wet-Look Gel

| | |
|---|---|
| 1,7 g | Sea Kem^{®} CM 611 (Carrageenan und Dextrose, FMC Corp.) |
| 2,5 g | Pure Thix^{®} TX 1442 (Polyether-1, Süd-Chemie, United Catalysts) |
| 10,0 g | Propylenglykol |
| 10,0 g | Ethanol |
| 0,3 g | Parfüm |
| 0,2 g | PEG-40 Hydrogenated Castor Oil |
| 0,2 g | PPG-1-PEG9 Lauryl Glycol Ether |
| 0,3 g | Methylparaben |
| 0,02 g | Mica |
| ad 100 g | Wasser |

## Patentansprüche

1. Haarbehandlungsmittel in Form eines festen und formstabilen Gels mit einem Gehalt an einer Kombination von
(A) 0,5 bis kleiner als 2 Gew.% kappa-Carrageenan und
(B) mindestens einem Zusatzstoff, ausgewählt aus
- amphiphilen Assoziativverdickern und
- mindestens 15 Gew.% mindestens eines ein- oder mehrwertigen C1- bis C5-Alkohols,
in einer wasserhaltigen Grundlage, wobei die Komponenten (A) und (B) in solchen Mengen enthalten sind, dass das Mittel bei Raumtemperatur in Form eines festen, formstabilen Gels vorliegt; ausgenommen einer Zusammensetzung bestehend aus 1,0 Gew,% Kappa-Carrageenan, 0,5 Gew.% Xanthan, 20,0 Gew.% Glycerin, 2,0 Gew.% Nylon-12 Pulver, 0,5 Gew.% Kaliumchlorid und der Rest bis 100 Gew.% Wasser.

2. Mittel nach Anspruch 1, **dadurch gekennzeichnet, dass** der Widerstand gegen Kompression unter Normbedingungen (20°C, 65% rel. Luftfeuchte) mindestens 0,15 N beträgt, gemessen durch Penetration des festen Gels mit einem zylindrischen Stempel eines Durchmessers von 8 mm, wobei die Penetration mit einer Geschwindigkeit von 0,5 mm/sec bis zu einer Kompressionstiefe von 1 mm erfolgt und anschließend der Stempel mit einer Geschwindigkeit von 0,5 mm/s wieder zurückgeführt wird.

3. Mittel nach einem der vorhergehenden Ansprüche, **dadurch gekennzeichnet, dass** das Mittel ein haarfestigendes Polymer ausgewählt aus anionischen, nichtionischen und amphoteren Polymeren enthält.

4. Mittel nach Anspruch 3, **dadurch gekennzeichnet, dass** das haarfestigende Polymer ausgewählt ist aus vernetzten oder unvernetzten Vinylacetat/Crotonsäure Copolymeren, partialveresterten Copolymeren zwischen Vinylmethylether und Maleinsäureanhydrid, Terpolymeren aus Acrylsäure, Alkylacrylat und N-Alkylacrylamid, Terpolymeren aus Vinylacetat, Crotonat und Vinylalkanoat, Copolymeren aus Acryl- oder Methacrylsäure und Acryl- oder Methacrylsäurealkylestern, Polyvinylpyrrolidonen und Vinylpyrrolidon/Vinylacetat Copolymeren sowie Polymeren aus Alkylacrylamid, Alkylaminoalkylmethacrylat und zwei oder mehr Monomeren bestehend aus Acrylsäure, Methacrylsäure oder deren Estern, wobei mindestens eines dieser Monomere eine Säuregruppe enthält.

5. Mittel nach einem der vorhergehenden Ansprüche, **dadurch gekennzeichnet, dass** das kappa-Carrageenan in einer Menge von mindestens 1 Gew.% bis kleiner 2 Gew.% und das haarfestigende Polymer in einer Menge von 0,5 bis 15 Gew.% vorliegt.

6. Mittel nach einem der vorhergehenden Ansprüche, **dadurch gekennzeichnet, dass** es zusätzlich mindestens einen die Festigkeit des Gels erhöhenden Stoff enthält, ausgewählt aus Calciumionen und Kaliumionen vorzugsweise in einer Menge von 0,2 bis 1 Gew.%.

7. Mittel nach einem der vorhergehenden Ansprüche, **dadurch gekennzeichnet, dass** es mindestens 15 Gew.% mindestens eines ein- oder mehrwertigen C1- bis C5-Alkohols enthält und frei von zusätzlichen Konservierungsmitteln ist.

8. Mittel nach einem der vorhergehenden Ansprüche, **dadurch gekennzeichnet, dass** der ein- oder mehrwertige Alkohol ausgewählt ist aus Ethanol, Isopropanol, Ethylenglykol, Glycerin und Propylenglykolen.

9. Mittel nach einem der vorhergehenden Ansprüche, **dadurch gekennzeichnet, dass** der amphiphile Assoziativverdicker ausgewählt ist aus hydrophob modifizierten Aminoplast-Polyether Copolymeren.

10. Mittel nach Anspruch 9, **dadurch gekennzeichnet, dass** der amphiphile Assoziativverdicker ausgewählt ist aus Polyether-1, PEG-180/ Octoxynol-40/TMMG Copolymeren und PEG-180/Laureth-50/TMMG Copolymeren.

11. Mittel nach einem der vorhergehenden Ansprüche, **dadurch gekennzeichnet, dass** es zusätzlich mindestens einen Zucker enthält.

12. Mittel nach Anspruch 11, **dadurch gekennzeichnet, dass** der Zucker ausgewählt ist aus Glukose, Galaktose, Fructose, Maltose, Lactose und Saccharose.

13. Mittel nach einem der vorhergehenden Ansprüche, **dadurch gekennzeichnet, dass** es in Form von Würfeln, Quadern, Kugeln, Eiern, Herzen, Buchstaben, Logos, Tierkörpern, Sternzeichen oder sonstigen dreidimensionalen geometrischen Formen vorliegt.

14. Verfahren zur Herstellung eines dreidimensionalen Formkörpers zur Haarbehandlung, wobei
- in eine Vorlage aus Stärkematerial mittels eines Stempels eine Form eingedrückt wird,
- in diese Form eine noch flüssige Gelmasse mit einer Zusammensetzung gemäß einem der Ansprüche 1 bis 12 eingefüllt wird,
- bis zur Verfestigung der Masse stehen gelassen wird und
- die Stärkemasse von dem verfestigten Formkörper entfernt wird.

## Claims

1. Hair treatment composition in the form of a solid, form-stable gel comprising a combination of
(A) 0.5 to less than 2% by weight of kappa-carrageenan and
(B) at least one additive selected from
- amphiphilic associative thickeners and
- at least 15% by weight of at least one C1 to C5 monoalcohol or polyalcohol,
in a hydrous base, components (A) and (B) being present in amounts such that the composition at room temperature is in the form of a solid, form-stable gel; with the exception of a composition consisting of 1.0% by weight kappa-carrageenan, 0.5% by weight xanthan, 20.0% by weight glycerol, 2.0% by weight Nylon-12 powder, 0.5% by weight potassium chloride, and water as the remainder up to 100% by weight.

2. Composition according to Claim 1, **characterized in that** the resistance to compression under standard conditions (20°C, 65% relative humidity) is at least 0.15 N, measured by penetration of the solid gel with a cylindrical piston of a diameter of 8 mm, the penetration taking place at a rate of 0.5 mm/sec until a compression depth of 1 mm is reached, after which the piston is withdrawn at a rate of 0.5 mm/s.

3. Composition according to either of the preceding claims, **characterized in that** the composition comprises a hair fixing polymer selected from anionic, non-ionic and amphoteric polymers.

4. Composition according to Claim 3, **characterized in that** the hair fixing polymer is selected from cross-linked or uncross-linked vinyl acetate/crotonic acid copolymers, partially esterified copolymers of vinyl methyl ether and maleic acid anhydride, terpolymers of acrylic acid, alkyl acrylate and N-alkylacrylamide, terpolymers of vinyl acetate, crotonate and vinyl alkanoate, copolymers of acrylic or methacrylic acid and acrylic or methacrylic acid alkyl esters, polyvinylpyrrolidones and vinylpyrrolidone/vinyl acetate copolymers, and also polymers of alkylacrylamide, alkylaminoalkylmethacrylate and two or more monomers consisting of acrylic acid, methacrylic acid or their esters, wherein at least one of said monomers contains an acid group.

5. Composition according to any one of the preceding claims, **characterized in that** the kappa-carrageenan is present in an amount of at least 1% by weight to less than 2% by weight and the hair fixing polymer is present in an amount from 0.5 to 15% by weight.

6. Composition according to any one of the preceding claims, **characterized in that** it further comprises at least one gel strength enhancer selected from calcium ions and potassium ions, preferably in an amount from 0.2 to 1% by weight.

7. Composition according to any one of the preceding claims, **characterized in that** it contains at least 15% by weight of at least one C1 to C5 monoalcohol or polyalcohol and is free from additional preservative ingredients.

8. Composition according to any one of the preceding claims, **characterized in that** the monoalcohol or polyalcohol is selected from ethanol, isopropanol, ethylene glycol, glycerol and propylene glycols.

9. Composition according to any one of the preceding claims, **characterized in that** the amphiphilic associative thickener is selected from hydrophobically modified aminoplast-polyether copolymers.

10. Composition according to Claim 9, **characterized in that** the amphiphilic associative thickener is selected from polyether-1, PEG-180/Octoxynol-40/TMMG copolymers and PEG-180/Laureth-50/TMMG copolymers.

11. Composition according to any one of the preceding claims, **characterized in that** it further comprises at least one sugar.

12. Composition according to Claim 11, **characterized in that** the sugar is selected from glucose, galactose, fructose, maltose, lactose and saccharose.

13. Composition according to any one of the preceding claims, **characterized in that** it is present in the format of cubes, parallelepipeds, spheres, eggs, hearts, letters, logos, animal body forms, stars or other three-dimensional geometric forms.

14. Process for making a three-dimensional body for hair treatment, where
- a shape is pressed into an initial charge of starch material, using a piston,
- a still fluid gel mass is introduced into this shape, the mass having a composition according to any one of Claims 1 to 12,
- the system is left to stand until the mass solidifies, and
- the starch mass is removed from the solidified body.

## Revendications

1. Composition de traitement capillaire, sous forme d'un gel solide et à stabilité dimensionnelle, ayant une teneur en une association de
(A) 0,5 à moins de 2 % en poids de kappa-carraghénane et
(B) au moins un additif choisi parmi
- des épaississants associatifs amphiphiles et
- au moins 15 % en poids d'au moins un alcool en C₁-C₅ monohydrique,
dans une base aqueuse, les composants (A) et (B) étant contenus en quantités telles que la composition à la température ambiante se trouve sous forme d'un gel solide à stabilité dimensionnelle ;
à l'exclusion d'une composition constituée de 1,0 % en poids de kappa-carraghénane, 0,5 % en poids de xanthane, 20,0 % en poids de glycérol, 2,0 % en poids de poudre de Nylon-12, 0,5 % en poids de chlorure de potassium et le reste jusqu'à 100 % consistant en eau.

2. Composition selon la revendication 1, **caractérisée en ce que** la résistance à la compression dans des conditions normalisées (20 °C, 65 % d'humidité relative de l'air) est d'au moins 0,15 N, mesurée par pénétration dans le gel solide d'un poinçon cylindrique d'un diamètre de 8 mm, la pénétration s'effectuant à une vitesse de 0,5 mm/s jusqu'à une profondeur de compression de 1 mm et le poinçon étant ensuite retiré à une vitesse de 0,5 mm/s.

3. Composition selon l'une quelconque des revendications précédentes, **caractérisée en ce que** la composition contient un polymère fixant les cheveux, choisi parmi des polymères anioniques, non ioniques et amphotères.

4. Composition selon la revendication 3, **caractérisée en ce que** le polymère fixant les cheveux est choisi parmi des copolymères acétate de vinyle/acide crotonique réticulés ou non réticulés, des copolymères entre l'éther vinylméthylique et l'anhydride maléique, partiellement estérifiés, des terpolymères d'acide acrylique, acrylate d'alkyle et N-alkylacrylamide, des terpolymères d'acétate de vinyle, crotonate et alcanoate de vinyle, des copolymères d'acide acrylique ou méthacrylique et d'acrylates ou méthacrylates d'alkyle, la polyvinylpyrrolidone et des copolymères vinylpyrrolidone/acétate de vinyle ainsi que des polymères d'alkylacrylamide, méthacrylate d'alkylaminoalkyle et de deux ou plus de deux monomères consistant en acide acrylique, acide méthacrylique ou leurs esters, au moins l'un de ces monomères comportant un groupe acide.

5. Composition selon l'une quelconque des revendications précédentes, **caractérisée en ce que** le kappa-carraghénane est présent en une quantité d'au moins 1 % en poids à moins de 2 % en poids et le polymère fixant les cheveux est présent en une quantité de 0,5 à 15 % en poids.

6. Composition selon l'une quelconque des revendications précédentes, **caractérisée en ce qu'**en outre elle contient au moins une substance augmentant la solidité du gel, choisie parmi des ions calcium et des ions potassium, de préférence en une quantité de 0,2 à 1 % en poids.

7. Composition selon l'une quelconque des revendications précédentes, **caractérisée en ce qu'**elle contient au moins 15 % en poids d'au moins un alcool monohydrique ou polyhydrique en C₁-C₅ et est exempte de conservateurs supplémentaires.

8. Composition selon l'une quelconque des revendications précédentes, **caractérisée en ce que** l'alcool monohydrique ou polyhydrique est choisi parmi l'éthanol, l'isopropanol, l'éthylèneglycol, le glycérol et les propylèneglycols.

9. Composition selon l'une quelconque des revendications précédentes, **caractérisée en ce que** l'épaississant associatif amphiphile est choisi parmi des copolymères aminoplaste-polyéther à modification hydrophobe.

10. Composition selon la revendication 9, **caractérisée en ce que** l'épaississant associatif amphiphile est choisi parmi des polyéthers-1, des copolymères PEG-180/octoxynol-40/TMMG et des copolymères PEG-180/laureth-50/TMMG.

11. Composition selon l'une quelconque des revendications précédentes, **caractérisée en ce qu'**en outre elle contient au moins un glucide.

12. Composition selon la revendication 11, **caractérisée en ce que** le glucide est choisi parmi le glucose, le galactose, le fructose, le maltose, le lactose et le saccharose.

13. Composition selon l'une quelconque des revendications précédentes, **caractérisée en ce qu'**elle se trouve sous la forme de cubes, de parallélépipèdes, de sphères, d'oeufs, de coeurs, de lettres, de logos, d'animaux, de signes du zodiaque ou d'autres formes géométriques tridimensionnelles.

14. Procédé pour la fabrication d'un corps moulé tridimensionnel pour le traitement capillaire, dans lequel
- on imprime un moule au moyen d'un poinçon dans un récipient en matériau à base d'amidon,
- on introduit dans ce moule une masse de gel encore fluide ayant une composition selon l'une quelconque des revendications 1 à 12,
- on laisse reposer jusqu'à la solidification de la masse et
- on élimine la masse d'amidon du corps moulé solidifié.
